# NEUE EUROPÄISCHE PATENTSCHRIFT

(11) **EP 0 784 046 B2**
(45) Veröffentlichungstag und Bekanntmachung des Hinweises auf die Entscheidung über den Einspruch: **06.04.2005**
(45) Hinweis auf die Patenterteilung: 28.03.2001
(21) Anmeldenummer: 97100374.4
(22) Anmeldetag: 10.01.1997
(51) Int. Cl.: C07C 57/04, C07C 51/43, C07C 51/48, C07C 51/25

(54) **Verfahren zur Herstellung von Acrylsäure und deren Estern**
Process for production of acrylic acid and their esters
Procédé pour la préparation de l'acide acrylique et leurs esters

(30) Priorität: 12.01.1996 DE 19600955
(43) Veröffentlichungstag der Anmeldung: 16.07.1997
(73) Patentinhaber: BASF AKTIENGESELLSCHAFT, 67056 Ludwigshafen (DE)
(72) Erfinder: Machhammer, Otto, Dr., 67281 Kirchheim (DE); Dockner, Toni, Dr., 67149 Meckenheim (DE); Engert, Gerd-Jürgen, Dr., 67069 Ludwigshafen (DE); Proll, Theo, Dr., 67098 Bad Dürkheim (DE)
(74) Vertreter: Isenbruck, Günter, Dr.

(56) Entgegenhaltungen:
- EP-A- 0 551 111
- EP-A- 0 616 998
- DE-A- 2 136 396
- DE-A- 2 164 767
- US-A- 3 473 238
- US-A- 4 230 888
- Distillation Operation, ed. H.Z. Kister, McGraw Hill, 1990, S. 583-599
- Grundoperationen chem. Verfahrenstechnik, W.R.A. Vauck et al, Deutscher Verlag für Grundstoffindustrie, 2000 S. 735

## Beschreibung

Die vorliegende Erfindung betrifft ein Verfahren und eine Vorrichtung zur Herstellung von Acrylsäure und deren Estern.

Acrylsäure ist eine bedeutende Grundchemikalie. Aufgrund ihrer sehr reaktionsfähigen Doppelbindung sowie der Säurefunktion eignet sie sich insbesondere als Monomer zur Herstellung von Polymerisaten. Von der hergestellten Menge an Acrylsäuremonomeren wird der größere Teil vor der Polymerisation - zu z.B. Klebstoffen, Dispersionen oder Lacken -verestert. Nur der kleinere Teil der hergestellten Acrylsäuremonomeren wird direkt - zu z.B. "Superabsorbern" - polymerisiert. Während im allgemeinen bei der direkten Polymerisation der Acrylsäure Monomere hoher Reinheit benötigt werden, sind die Anforderungen an die Reinheit der Acrylsäure nicht so hoch, wenn diese vor der Polymerisation verestert wird.

Es ist allgemein bekannt, daß Acrylsäure durch heterogen katalysierte Gasphasenoxidation von Propen mit molekularem Sauerstoff an im festen Aggregatzustand befindlichen Katalysatoren bei Temperaturen zwischen 200 und 400°C zweistufig über Acrolein hergestellt werden kann (vgl. z.B. DE-A 19 62 431, DE-A 29 43 707, DE-PS 1 205 502, EP-A 257 565, EP-A 253 409, DE-AS 22 51 364, EP-A 117 146, GB-PS 1 450 986 und EP-A 293 224). Hierbei werden oxidische Mehrkomponenten-Katalysatoren z.B. auf der Basis von Oxiden der Elemente Molybdän, Chrom, Vanadium oder Tellur eingesetzt.

Aus DE-PS 21 36 396 ist bekannt, die Acrylsäure aus den bei der katalytischen Oxidation von Propen bzw. Acrolein erhaltenen Reaktionsgasen durch Gegenstromabsorption mit einem Gemisch aus 75 Gew.-% Diphenylether und 25 Gew.-% Diphenyl abzutrennen. Weiterhin ist aus DT-A 24 49 7800 das Abkühlen des heißen Reaktionsgases durch Teilverdampfen des Lösungsmittels in einem Direktkondensator (Quenchapparat) vor der Gegenstromabsorption bekannt. Problematisch ist hierbei sowie bei weiteren Verfahrensschritten der Anfall von Feststoffen in den Apparaten, der die Anlagenverfügbarkeit reduziert. Gemäß DE-A 43 08 087 kann dieser Feststoffanfall dadurch reduziert werden, indem man dem relativ unpolaren Lösungsmittelgemisch aus Diphenylether und Diphenyl ein polares Lösungsmittel, wie Dimethylphthalat in einer Menge von 0,1 bis 25 Gew.-% zufügt.

Neben der oben beschriebenen Absorption des die Acrylsäure enthaltenden Reaktionsprodukts in ein hochsiedendes Lösungsmittelgemisch sehen andere bekannte Verfahren eine Totalkondensation von Acrylsäure und des weiterhin bei der katalytischen Oxidation entstehenden Reaktionswassers vor. Dabei entsteht eine wäßrige Acrylsäurelösung, die über Destillation mit einem azeotropen Mittel (vgl. DE-PS 34 29 391, JA 11 24 766, JA 71 18 766, JA 71 18 966-R, JA 71 18968-R, JA-72 41 885) oder über ein Extraktionsverfahren (vgl. DE-OS 2 164 767, J5 81 40-039, J4 80 91 013) weiter aufgearbeitet werden kann. In EP-A 551 111 wird das mittels katalytischer Gasphasenoxidation hergestellte Gemisch von Acrylsäure und Nebenprodukten mit Wasser in einem Absorptionsturm in Berührung gebracht und die erhaltene wäßrige Lösung in Anwesenheit eines Lösungsmittels, das mit polaren Leichtsiedern wie Wasser oder Essigsäure ein Azeotrop bildet, destilliert. DE-PS 23 23 328 beschreibt die Abtrennung von Acrylsäure aus einer wäßrigen Acrylsäure-Veresterungsablauge oder einer wäßrigen Acrlysäure-Lösung, wie sie bei der Acrylsäure-Herstellung durch Oxidation von Propen oder Acrolein entsteht, durch Extraktion mit einem speziellen Gemisch organischer Lösungsmittel.

DE-A-21 64 767 betrifft ein Verfahren zur Reinigung von Acrylsäure. Dabei wird Acrylsäure durch katalytische Gasphasenoxidation von Propen oder Acrolein erhalten. Das erhaltene Reaktionsprodukt wird kondensiert und mit einem Extraktionsmittel extrahiert. Dabei wird ein Extraktionsmittel eingesetzt, das ein Acrylsäure zu Essigsäure Verteilungskoeffizientenverhältnis von über 1 hat. Derartige Lösungsmittel sind beispielsweise Ethylacetat, n-Propylacetat, Isopropylacetat, n-Butylacetat, Isobutylacetat, sec-Butylacetat, Methylpropionat, Ethylpropionat, Methylacrylat oder Ethylacrylat sowie Ketone wie Methylisobutylketon. Durch Wahl des Lösungsmittels wird erreicht, daß im Reaktionsprodukt enthaltene Essigsäure als flüssiger Extraktionsrückstand anfällt und vom Acrylsäure/Extraktions-Gemisch abgetrennt werden kann. Der Extrakt enthält Acrylsäure und noch einen geringeren Anteil an Essigsäure. Er wird sodann einer Lösungsmittelabtrennkolonne zugeführt, wo das in dem Extrakt enthaltene Wasser und das Lösungsmittel abgetrennt werden. Dabei wird eine flüssige Acrylsäure/Essigsäure-Mischung als Sumpf erhalten. Diese Mischung wird in eine Essigsäureabtrennkolonne eingeführt, in der die Essigsäure von der Acrylsäure abdestilliert wird.

EP-A-0 616 998 betrifft ein Verfahren und eine Vorrichtung zur Reinigung von Acrylsäure in einer Kombination von dynamischer und statischer Kristallisation in mehreren Stufen, wobei der Rückstand der dynamischen Kristallisation durch die statische Kristallisation weiter gereinigt wird und die dabei gewonnene Acrylsäure wieder der dynamischen Kristallisation zugeführt wird. Hierdurch ist insbesondere eine Abtrennung von Propionsäure von der Acrylsäure möglich.

Unabhängig von der Art der oben dargestellten Verfahren zur Herstellung der Acrylsäure reicht im allgemeinen die damit erzielte Qualität nicht aus, um daraus verkaufsfähige Produkte polymerisieren zu können. Bei einer Weiterverarbeitung zu Polyacrylsäure stören insbesondere (bei der katalytischen Gasphasenoxidation entstehende) Essigsäure, Propionsäure und Aldehyde. Es ist bekannt, Aldehyde durch eine chemische Vorbehandlung mit AGHC (Aminoguanidinhydrogencarbonat) oder NH₂NH₂ (vgl. z.B. EP-A 270 999, PT-78-759-A, PT 81 876-A, US-37 25 208-S) und weiteren destillativen Aufarbeitungsschritten (Leichtsieder-Destillation und Acrylsäure-Rein-Destillation) zu entfernen. Auf diese Weise gereinigte Acrylsäure eignet sich z.B. zur Herstellung von "Superabsorbern". Weiterhin ist bekannt, Essigsäure nach der Veresterung destillativ aus den Acrylaten abzutrennen.

Selbst wenn die Acrylsäure nicht als direkter Ausgangsstoff für die Erzeugung von Polyacrylsäure eingesetzt, sondern vor der Weiterverarbeitung verestert wird, was für den überwiegenden Teil der weltweit hergestellten Rohsäure zutrifft, stören die oben angesprochenen Nebenkomponenten Essigsäure, Propionsäure und Aldehyde.

Somit stören praktisch bei allen Weiterverarbeitungsverfahren von Acrylsäure die gleichen Nebenkomponenten, nämlich Essigsäure, Propionsäure und Aldehyde. Eine Destillation von Acrylsäure ist wegen der hohen Temperaturbeanspruchung und der damit verbundenen starken Neigung der Acrylsäure zur Bildung von Dimeren, Oligomeren oder Polymerisaten problematisch. Außerdem können hierbei ähnlich siedende Nebenkomponenten, insbesondere Propionsäure, nicht abgetrennt werden (Siedetemperatur von Acrylsäure 141,6°C, Siedetemperatur von Propionsäure 140,9°C)

Somit bestand die Aufgabe der vorliegenden Erfindung darin, ein Verfahren zur Herstellung von Acrylsäure oder deren Estern zu schaffen, bei dem die oben genannten störenden Nebenkomponenten entfernt werden und das es erlaubt, je nach Erfordernis sehr flexibel Acrylsäure unterschiedlicher Qualität zur Verfügung zu stellen.

Diese Aufgabe wird gelöst durch ein Verfahren zur Herstellung von Acrylsäure und/oder deren Estern, wobei das Verfahren folgende Stufen in der folgenden Reihenfolge umfaßt:
(a) katalytische Gasphasenoxidation von Propen und/oder Acrolein zu Acrylsäure unter Erhalt eines gasförmigen, die Acrylsäure enthaltenden Reaktionsprodukts,
(b) Absorption des Reaktionsprodukts mit einem Lösungsmittel,
   (bl) das mit Reaktionsprodukt beladene Lösungsmittel wird einer Desorption zur Abtrennung von leicht siedenden Nebenkomponenten unterzogen,
(c) Destillation des mit Acrylsäure beladenen Lösungsmittelstroms aus (bl) in einer Kolonne unter Erhalt einer Roh-Acrylsäure, die durch Seitenabzug entnommen wird, und des Lösungsmittels im Sumpf,
(d) Reinigung der Roh-Acrylsäure durch Kristallisation und
(e) gegebenenfalls Veresterung der kristallisierten Acrylsäure.

Die Erfindung betrifft auch eine Vorrichtung zur Herstellung von Acrylsäure, umfassend in der folgenden Reihenfolge
- wenigstens einen Reaktor für eine katalytische Gasphasenreaktion,
- eine Absorptionskolonne,
- eine Desorptionskolonne,
- eine Destillationskolonne, mit Seitenabzug, durch den die Acrylsäure abgezogem wird,
- wenigstens einen dynamischen Kristallisator und wenigstens einen statischen Kristallisator und
- wenigstens einen Behälter zur Zwischenlagerung.

Weiterhin betrifft die Erfindung eine Vorrichtung zur Durchführung des erfindungsgemäßen Verfahrens. Bevorzugte Ausführungsformen der Erfindung sind in den zugehörigen Unteransprüchen definiert.

### Stufe (a):

Erfindungsgemäß kann die katalytische Gasphasenreaktion von Propen und/oder Acrolein mit molekularem Sauerstoff zu Acrylsäure nach bekannten Verfahren, insbesondere wie sie in den oben genannten Druckschriften beschrieben sind, erfolgen. Vorzugsweise wird hierbei bei Temperaturen zwischen 200 und 400°C gearbeitet. Vorzugsweise werden als heterogene Katalysatoren oxidische Mehrkomponenten-Katalysatoren auf der Basis der Oxide von Molybdän, Chrom, Vanadium und/oder Tellur eingesetzt.

Die Umsetzung von Propen zu Acrylsäure ist stark exotherm. Das Reaktionsgas, das neben den Edukten und Produkten vorteilhafterweise ein Verdünnungsgas, z.B. Kreisgas (siehe unten), Luftstickstoff und/oder Wasserdampf enthält, kann daher nur einen kleinen Teil der Reaktionswärme aufnehmen. Obwohl die Art der verwendeten Reaktoren an sich keiner Beschränkung unterliegt, werden meist Rohrbündelwärmetauscher verwendet, die mit dem Oxidationskatalysator gefüllt sind, da bei diesen der überwiegende Teil der bei der Reaktion freiwerdenden Wärme durch Konvektion und Strahlung an die gekühlten Rohrwände abgeführt werden kann.

In Stufe (a) wird jedoch nicht reine Acrylsäure, sondern ein gasförmiges Gemisch erhalten, das neben der Acrylsäure als Nebenkomponenten im wesentlichen nicht umgesetztes Acrolein und/oder Propen, Wasserdampf, Kohlenmonoxid, Kohlendioxid, Stickstoff, Sauerstoff, Essigsäure, Propionsäure Formaldehyd, weitere Aldehyde und Maleinsäureanhydrid enthalten kann.

Üblicherweise enthält das Reaktionsproduktgemisch, jeweils bezogen auf das gesamte Reaktionsgemisch, von 0,05 bis 1 Gew.-% Propen und 0,05 bis 1 Gew.-% Acrolein, 0,01 bis 2 Gew.-% Propan, 1 bis 20 Gew.-% Wasserdampf, 0,05 bis 15 Gew.-% Kohlenoxide, 10 bis 90 Gew.-% Stickstoff, 0,05 bis 5 Gew.-% Sauerstoff, 0,05 bis 2 Gew.-% Essigsäure, 0,01 bis 2 Gew.-% Propionsäure, 0,05 bis 1 Gew.-% Formaldehyd, 0,05 bis 2 Gew.-% Aldehyde sowie 0,01 bis 0,5 Gew.-% Maleinsäureanhydrid.

### Stufe (b)

In Stufe (b) wird die Acrylsäure und ein Teil der Nebenkomponenten aus dem Reaktionsgas durch Absorption mit einem Lösungsmittel abgetrennt. Erfindungsgemäß eignen sich als Lösungsmittel insbesondere alle hochsiedenden Lösungsmittel, vorzugsweise Lösungsmittel mit einem Siedepunkt über 160 °C. Besonders geeignet ist ein Gemisch aus Diphenylether und Biphenyl, insbesondere das im Handel erhältliche Gemisch aus 75 Gew.-% Diphenylether und 25 Gew.-% Biphenyl.

Vorliegend bezeichnen die Begriffe Hoch- oder Schwersieder, Mittelsieder und Leichtsieder sowie entsprechend adjektivisch gebrauchte Begriffe Verbindungen, die einen höheren Siedepunkt als die Acrylsäure besitzen (Hochsieder) bzw. solche, die in etwa den gleichen Siedepunkt wie Acrylsäure besitzen (Mittelsieder) bzw. solche, die einen niedrigeren Siedepunkt als Acrylsäure besitzen (Leichtsieder).

Vorteilhafterweise wird das aus Stufe (a) erhaltene heiße Reaktionsgas durch Teilverdampfen des Lösungsmittels in einem geeigneten Apparat, z.B. einem Direktkondensator oder Quenchapparat, vor der Absorption abgekühlt. Hierfür eignen sich u.a. Venturiwäscher, Blasensäulen oder Sprühkondensatoren.

Dabei kondensieren die schwersiedenden Nebenkomponenten des Reaktionsgases aus Stufe (a) in das nicht verdampfte Lösungsmittel. Außerdem ist die Teilverdampfung des Lösungsmittels ein Reinigungsschritt für das Lösungsmittel. In einer bevorzugten Ausführungsform der Erfindung wird ein Teilstrom des nicht verdampften Lösungsmittels, vorzugsweise 1 bis 10 % des der Absorptionskolonne zugeführten Massenstromes, abgezogen und einer Lösungsmittelreinigung unterworfen. Hierbei wird das Lösungsmittel überdestilliert und zurück bleiben die schwersiedenden Nebenkomponenten, die - bei Bedarf weiter eingedickt - entsorgt, z.B. verbrannt, werden können. Diese Lösungsmitteldestillation dient der Vermeidung einer zu hohen Konzentration an Schwersieder im Lösungsmittelstrom.

Die Absorption erfolgt in einer Gegenstromabsorptionskolonne, die vorzugsweise mit Ventil- oder Dualflowböden bestückt ist, die von oben mit (nicht verdampftem) Lösungsmittel beaufschlagt wird. Das gasförmige Reaktionsprodukt und gegebenenfalls verdampftes Lösungsmittel werden von unten in die Kolonne eingeleitet und anschließend auf Absorptionstemperatur abgekühlt. Die Abkühlung erfolgt vorteilhafterweise durch Kühlkreise, d.h. erwärmtes Lösungsmittel wird aus der Kolonne abgezogen, in Wärmetauschern abgekühlt und wieder an einer Stelle oberhalb der Abzugsstelle der Kolonne zugeführt. In diese Lösungsmittelkühlkreise kondensieren neben der Acrylsäure leicht-, schwer- und mittelsiedende Nebenkomponenten sowie verdampftes Lösungsmittel. Sobald der Reaktionsgasstrom auf die Absorptionstempteratur abgekühlt ist, erfolgt die eigentliche Absorption. Dabei wird der im Reaktionsgas verbliebene Rest der Acrylsäure absorbiert sowie ein Teil der leichtsiedenden Nebenkomponenten.

Das verbleibende, nicht absorbierte Reaktionsgas von Stufe (a) wird weiter abgekühlt, um den kondensierbaren Teil der leichtsiedenden Nebenkomponenten davon, insbesondere Wasser, Formaldehyd und Essigsäure, durch Kondensation abzutrennen. Dieses Kondensat wird im folgenden Sauerwasser genannt. Der verbleibende Gasstrom, im folgenden Kreisgas genannt, besteht überwiegend aus Stickstoff, Kohlenoxiden und nicht umgesetzten Edukten. Vorzugsweise wird dieser teilweise wieder als Verdünnungsgas den Reaktionsstufen zugeführt.

Aus dem Sumpf der in Stufe (b) eingesetzten Kolonne wird ein mit Acrylsäure, schwer- und mittelsiedenden Nebenkomponenten sowie einem geringen Anteil an leichtsiedenden Nebenkomponenten beladener Lösungsmittelstrom abgezogen und einer Desorption unterzogen. Vorteilweise wird diese in einer Kolonne, die vorzugsweise mit Ventil- oder Dualflowböden aber auch mit Füllkörpem oder geordneten Packungen bestückt sein kann, in Anwesenheit eines sog. Strippoder Abstreifgases durchgeführt. Bei dem Strippgas kann jedes inerte Gas oder Gasgemisch verwendet werden, vorzugsweise wird ein Gasgemisch von Luft und Stickstoff oder Kreisgas verwendet, da dieses in Stufe (a) bei Durchführung einer Verdampfung eines Teils des Lösungsmittels anfällt. Bei der Desorption wird der größte Teil der Leichtsieder mit einem Teil des Kreisgases, das vor Stufe (a) entnommen wird, aus dem beladenen Lösungsmittel gestrippt. Da hierbei auch größere Mengen an Acrylsäure mitgestrippt werden, wird zweckmäßigerweise dieser Strom, der im folgenden Strippkreisgas genannt wird, aus wirtschaftlichen Gründen nicht verworfen, sondern rezirkuliert, z. B. in die Stufe, in der die Teilverdampfung des Lösungsmittels erfolgt, oder in die Absorptionskolonne. Da das Strippgas ein Teil des Kreisgases ist, enthält es selbst noch nennenswerte Mengen an Leichtsiedern. Die Leistung der zur Desorption verwendeten Kolonne läßt sich erhöhen, wenn man die Leichtsieder vor dem Einleiten in die Kolonne aus dem Strippgas entfernt. Zweckmäßigerweise wird dieses verfahrenstechnisch in der Art durchgeführt, indem man das Strippgas mit in der unten beschriebenen Stufe (c) aufgearbeitetem Lösungsmittel in einer Gegenstromwaschkolonne reinigt.

Aus dem Sumpf der zur Desorption verwendeten Kolonne wird dann ein nahezu leichtsiederfreier, mit Acrylsäure beladener Lösungsmittelstrom abgezogen.

### Stufe (c):

In Verfahrensstufe (c) wird die Acrylsäure zusammen mit den mittelsiedenden Komponenten sowie dem letzten Rest an leichtsiedenden Nebenkomponenten vom Lösungsmittel abgetrennt. Diese Trennung erfolgt mittels Destillation, wobei grundsätzlich jede Destillationskolonne mit Seitenabzug, durch den Acrylsäure abgezegen wird, verwendet werden kann. Vorzugsweise wird eine Kolonne mit Siebböden, z.B. Dual-Flow-Böden oder Querstromsiebböden aus Metall verwendet. Im Auftriebsteil der Kolonne wird die Acrylsäure vom Lösungsmittel und den mittelsiedenden Nebenkomponenten, wie Maleinsäureanhydrid, frei destilliert. Um den Leichtsiederanteil in der Acrylsäure zu reduzieren, wird der Auftriebsteil der Kolonne verlängert und die Acrylsäure als Seitenabzug aus der Kolonne abgezogen. Diese Acrylsäure wird im folgenden, unabhängig von ihrer Reinheit, Roh-Acrylsäure genannt.

Am Kopf der Kolonne wird dann nach einer Partialkondensation ein an Leichtsiedern reicher Strom abgezogen. Da dieser Strom aber noch Acrylsäure enthält, wird er vorteilhafterweise nicht verworfen, sondern der Absorptionsstufe (b) rückgeführt.

Aus dem Sumpf der Kolonne wird das an Leichtsiedern freie und nahezu an Acrylsäure freie Lösungsmittel abgezogen und vorzugsweise zum überwiegenden Teil der Gegenstromwaschkolonne zugeführt, in der das Strippgas von Stufe (b) gereinigt wird, um die Leichtsieder aus dem Strippgas zu waschen. Anschließend wird das nahezu Acrylsäure-freie Lösungsmittel der Absorptionskolonne zugeführt.

In einer bevorzugten Ausführungsform der Erfindung wird das Sauerwasser, das noch Acrylsäure gelöst enthalten kann, mit einem kleinen Teilstrom des nahezu Acrylsäure-freien Lösungsmittels extraktiv behandelt. Bei dieser Sauerwasser-Extraktion wird ein Teil der Acrylsäure in das Lösungsmittel extrahiert und somit aus dem Sauerwasser zurückgewonnen. Das Sauerwasser extrahiert im Gegenzug die polaren mittelsiedenden Komponenten aus dem Lösungsmittelstrom und vermeidet damit eine Zunahme dieser Komponenten im Lösungsmittelkreislauf. Der erhaltene Sauerwasserstrom aus Leicht- und Mittelsieder kann, was insbesondere bei Vorhandensein von Umweltschutzauflagen erforderlich ist, eingeengt werden. Damit können auch die hohen Auflagen, wie sie in den Vereinigten Staaten anzutreffen sind ("Hazardous Waste Law"), erfüllt werden.

Die in Stufe (c) erhaltene Roh-Acrylsäure enthält, jeweils bezogen auf die Roh-Acrylsäure, vorzugsweise 98 bis 99,8 Gew.-%, insbesondere 98,5 bis 99,5 Gew.-%, Acrylsäure und 0,2 bis 2 Gew.-%, insbesondere 0,5 bis 1,5 Gew.-%, Verunreinigungen, wie z.B. Essigsäure, Aldehyde und Maleinsäureanhydrid.

### Stufe (d):

In Stufe (d) wird die in Stufe (c) erhaltene Roh-Acrylsäure durch Kristallisation, vorzugsweise mittels fraktionierter Kristallisation durch eine Kombination von dynamischer und statischer Kristallisation weiter gereinigt. Die Art der verwendeten dynamischen oder statischen Kristallisation unterliegt hierbei keiner besonderen Beschränkung.

Bei der statischen Kristallisation (z.B. US 3 597 164 und FR 2 668 946) wird die flüssige Phase nur durch freie Konvektion bewegt, während bei der dynamischen Kristallisation die flüssige Phase durch erzwungene Konvektion bewegt wird. Letzteres kann durch eine erzwungene Strömung in voll durchströmten Apparaten (vgl. DE 26 06 364) oder durch die Aufgabe eines Riesel- oder Fallfilms auf eine gekühlte Wand (DT 1 769 123 und EP 218 545) erfolgen.

Erfindungsgemäß bevorzugt wird die zu reinigende Roh-Acrylsäure in flüssiger Phase in den Kristallisationsapparat eingefüllt und anschließend an den gekühlten Flächen eine feste Phase ausgefroren, die sich in ihrer Zusammensetzung von der eingefüllten flüssigen Phase unterscheidet. Nachdem ein bestimmter Anteil der eingesetzten Acrylsäure ausgefroren ist (zweckmäßigerweise zwischen 50 - 80 %, insbesondere 60 - 70%), wird die verbleibende flüssige Restphase abgetrennt. Vorteilhafterweise erfolgt dies durch einfaches Abfließenlassen oder Abpumpen der Restphase. Dem Kristallisationsschritt können sich weitere Reinigungsschritte wie das sog. Waschen der Kristallschicht (vgl. DE 3 708 709) oder das sog. Schwitzen, d.h. ein partielles Abschmelzen verunreinigter Kristallbereiche, anschließen. Zweckmäßigerweise wird dem Kristallisationsschritt ein Schwitzschritt angeschlossen, wenn die gesamte Reinigungswirkung einer Stufe verbessert werden soll.

Die dynamische und statische Kristallisation können jeweils ein- oder mehrstufig durchgeführt werden. Mehrstufige Verfahren werden hierbei zweckmäßigerweise nach dem Gegenstromprinzip betrieben, bei dem nach der Kristallisation in jeder Stufe das Kristallisat vom Rückstand abgetrennt wird und dieses Kristallisat der jeweiligen Stufe mit dem nächsthöheren Reinheitsgrad zugeführt wird, während der Kristallisationsrückstand der jeweiligen Stufe mit dem nächst niedrigen Reinheitsgrad zugeführt wird. Üblicherweise werden alle Stufen, die ein Kristallisat erzeugen, das reiner ist als die zugeführte Roh-Acrylsäurelösung Reinigungsstufen genannt und alle anderen Stufen Abtriebsstufen genannt.

Vorteilhafterweise werden mehrere Stufen im selben Kristallisationsapparat sequenziell gefahren, wobei die einzelnen Stufenfraktionen (Kristallisat und Restschmelze bzw. Kristallisationsrückstand) in Behältern zwischengepuffert werden. Um die Produktverluste möglichst niedrig zu halten, wird eine hohe Kristallisationsausbeute angestrebt. Jedoch wird die maximal erreichbare Ausbeute durch die Lage der eutektischen Punkte zwischen Acrylsäure und ihren Nebenkomponenten begrenzt. Wird während der Kristallisation eine eutektische Zusammensetzung erreicht, so findet keine Stofftrennung mehr zwischen Acrylsäure und der entsprechenden Verunreinigung/Nebenkomponente statt. Es wurde in Kristallisationsversuchen mit Acrylsäure unterschiedlicher Reinheit gefunden, daß sich schon bei Verunreinigungskonzentrationen unter der eutektischen Zusammensetzung bei der dynamischen Kristallisation nurmehr schlecht haftende Kristallschichten ausbilden. Es wird deshalb vorgeschlagen, wie in EP 0 616 998 beschrieben, die Restschmelze aus der dynamischen Kristallisation in einer statischen Kristallisation weiter aufzuarbeiten. Dort wird aufgrund der sehr langsam ablaufenden Schichtbildung eine gute Schichthaftung mit guter Reinigungswirkung erreicht. Die statischen Stufen werden entsprechend dem oben beschriebenen Gegenstromprinzip in den Gesamtprozeß integriert, d.h. das Kristallisat mit der höchsten Reinheit aus der statischen Kristallisation wird der Stufe, in der das Kristallisat mit der niedrigsten Reinheit nach dem dynamischen Kristallisationsprinzip hergestellt wird, zugeführt und die Restschmelze aus der dynamischen Kristallisation mit dem niedrigsten Reinheitsgrad wird der Stufe zugeführt, in der statisch das reinste Kristallisat erzeugt wird.

Vorteilhafterweise wird die dynamische Kristallisation in einem voll durchströmten Apparat oder mittels eines Fall- oder Rieselfilms durchgeführt. Diese beiden Methoden sind besonders schnell und effizient. Die Anzahl der erforderlichen Kristallisationsstufen bestimmt sich nach dem Grad der gewünschten Reinheit und läßt sich leicht ermitteln. Zweckmäßigerweise friert man vor Beginn der Kristallisation eine Impfschicht an.

### Stufe (e):

Falls gewünscht, wird die in Stufe (d) erhaltene Rein-Acrylsäure nach bekannten Methoden verestert.

Das Verfahren und eine Vorrichtung werden anhand der Zeichnung beschrieben, die eine bevorzugte Ausführungsform der Erfindung darstellt. Darin zeigt:
- Fig. 1: ein Blockschaltbild des erfindungsgemäßen Verfahrens,
- Fig. 2: die Verschaltung der Apparate im erfindungsgemäßen Verfahren.

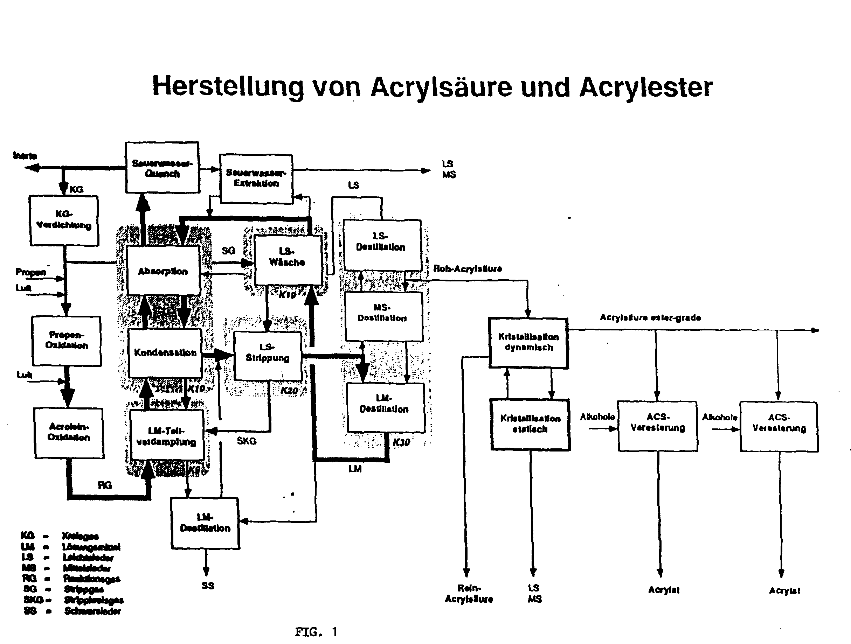

Gemäß Fig. 1 wird nach der Verdichtung des Kreisgases, das im wesentlichen aus Stickstoff, Kohlenoxiden und nicht umgesetzten Edukten besteht, dieses zusammen mit Propen und Luft einem Reaktor zugeführt, in dem die heterogen katalysierte Oxidation von Propen zu Acrolein stattfindet. Dem dabei entstehenden Zwischenreaktionsgas wird weiterhin Luft zugeführt, um im zweiten Reaktor die heterogen katalysierte Oxidation von Acrolein durchzuführen.

Das entstehende heiße, die Acrylsäure enthaltende, gasförmige Reaktionsprodukt wird durch Teilverdampfen des Lösungsmittels in einem Direktkondensator K9 vor der Absorption abgekühlt. Hierbei kondensieren die schwersiedenden Nebenkomponenten des Reaktionsproduktes in das nichtverdampfte Lösungsmittel. Ein Teilstrom aus dem Direktkondensator K9 wird einer Lösungsmitteldestillation unterzogen, wobei das Lösungsmittel überdestilliert wird und die schwersiedenden Nebenkomponenten zurückbleiben. Letztere können weiter eingedickt und entsorgt, z.B. verbrannt, werden.

Die Kolonne K10 wird von oben mit (nichtverdampftem) Lösungsmittel beaufschlagt, während das verdampfte Lösungsmittel und das gasförmige Reaktionsprodukt von unten in die Kolonne K10 eingeleitet und anschließend auf Absorptionstemperatur abgekühlt werden. Die Abkühlung erfolgt durch Kühlkreise (nicht gezeigt). In diese Kühlkreise kondensieren sowohl das verdampfte Lösungsmittel als auch die Acrylsäure sowie alle schwer- und mittelsiedenden Nebenkomponenten. Nachdem der gesamte Reaktionsgasstrom auf die Absorptionstemperatur abgekühlt ist, erfolgt die eigentliche Absorption. Dabei wird der im Reaktionsgas verbleibende Rest der Acrylsäure absorbiert sowie ein Teil der leichtsiedenden Nebenkomponenten. Anschließend wird das nicht absorbierte, verbleibende Reaktionsgas weiter abgekühlt, um den kondensierbaren Teil der leichtsiedenden Nebenkomponenten aus dem Gasstrom abzutrennen, in Fig. 1 gezeigt als Sauerwasser-Quench. Im folgenden wird deshalb dieses Kondensat Sauerwasser genannt. Der verbliebene Gasstrom, das Kreisgas, kann nun teilweise wieder als Verdünnungsgas den Reaktionsstufen wie in Fig. 1 gezeigt, zugeführt werden.

Aus dem Sumpf der Kolonne K10 wird das mit Acrylsäure und Nebenkomponenten beladene Lösungsmittel abgezogen und der Desorptionskolonne K20 zugeführt. In dieser werden der größte Teil der Leichtsieder mit einem Teil des Kreisgases, das vor den Oxidationsstufen entnommen wird, aus dem beladenen Lösungsmittel gestrippt. Da hierbei auch größere Mengen an Acrylsäure mitgestrippt werden, wird dieser Strom z.B. wieder in den Direktkondensator K9 rezirkuliert.

Zur Erhöhung der Desorptionsleistung der Kolonne K20 werden die Leichtsieder, die im Strippgas enthalten sind, vor dem Einleiten in die Kolonne K20 entfernt. Verfahrenstechnisch erfolgt dies zweckmäßigerweise dadurch, daß das Strippgas mit aufgearbeitetem Lösungsmittel aus der unten näher beschriebenen Kolonne K30 in einer Gegenstromwaschkolonne K19 gereinigt wird.

Im nächsten Verfahrensschritt wird aus dem Sumpf der Desorptionskolonne K20 ein nahezu leichtsiederfreier, mit Acrylsäure beladener Lösungsmittelstrom abgezogen und der Destillationskolonne K30, bei der es sich vorzugsweise um eine Siebbodenkolonne handelt, zugeführt. In den Sumpf der Kolonne K30 kondensiert das schwersiedende Lösungsmittel und die mittelsiedenden Nebenkomponenten, z.B. Maleinsäureanhydrid. Da die am Kopf der Kolonne K30 abgezogene Acrylsäure noch nennenswerte Mengen an leichtsiedenden Nebenkomponenten enthält, reduziert man diesen Leichtsiederanteil dadurch, daß man den Auftriebsteil der Kolonne K30 weiter verlängert und die Acrylsäure als Seitenabzug aus der Kolonne abzieht. Diese Acrylsäure wird Roh-Acrylsäure genannt.

Der am Kopf der Destillationskolonne K30 abgezogene Leichtsieder-reiche Strom wird, da er noch Acrylsäure enthält, zweckmäßigerweise wieder in die Absorptionskolonne K10 zurückgeführt.

Das aus dem Sumpf der Destillationskolonne K30 abgezogene, Leichtsiederfreie und fast Acrylsäure-freie Lösungsmittel wird zum überwiegenden Teil der Gegenstromwaschkolonne K19 zugeführt, um, wie bereits oben erwähnt, die Leichtsieder aus dem Strippgasstrom, der in die Desorptionskolonne K20 führt, zu waschen. Anschließend wird das nahezu Acrylsäure-freie Lösungsmittel der Absorptionskolonne K10 zugeführt. Mit einem kleinen Teilstrom des nahezu Acrylsäure-freien Lösungsmittels aus dem Sumpf der Destillationskolonne K30 wird das Sauerwasser, das noch Acrylsäure gelöst enthält, extraktiv behandelt. Bei dieser Sauerwasser-Extraktion wird ein Teil der Acrylsäure aus dem Sauerwasser zurückgewonnen, während im Gegenzug das Sauerwasser alle polaren Komponenten aus dem Lösungsmittelteilstrom extrahiert. Das hierbei entstehende Sauerwasser kann vorverdampft und anschließend verbrannt werden.

Die aus dem Seitenabzug der Destillationskolonne K30 gewonnene Roh-Acrylsäure wird anschließend einer dynamischen und statischen Kristallisation unterzogen. In Abhängigkeit von den gewünschten Reinheitsanforderungen an die Acrylsäure kann es auch genügen, wenn nur eine dynamische Kristallisation durchgeführt wird. Die dynamische Kristallisation wird entweder mit einem voll durchströmten Rohr oder mittels eines Fallfilms durchgeführt. Die Anzahl der Kristallisationsstufen variiert in Abhängigkeit von der gewünschten Reinheit der Acrylsäure. Vorzugsweise ist der dynamische Kristallisator als Wärmetauscher ausgebildet. Falls gewünscht, wird die erhaltene Rein-Acrylsäure dann mit Alkoholen zu den gewünschten Acrylaten verestert.

Das erfindungsgemäße Verfahren bietet somit folgende Vorteile:
- geringere Ausbeuteverluste durch den kombinierten Einsatz von dynamischer und statischer Kristallisation;
- bessere Säure- bzw. Ester-Qualität, da die Propionsäure sich durch Kristallisation abtrennen läßt. Propionsäure wird bei der Acrylesterherstellung zur übelriechenden Propionaten verestert und würde deshalb in jedem Fall in den Endprodukten stören;
- kein Zusatz eines chemischen Reagenzes zur Entfernung von Aldehyden notwendig;
- keine Acetatdestillation bei Weiterverarbeitung der Säure zu Ester notwendig;
- niedrigerer Alkoholverbrauch bei der Veresterung.

Die Erfindung wird anhand der folgenden Beispiele, die bevorzugte Ausführungsformen der Erfindung darstellen, näher erläutert.

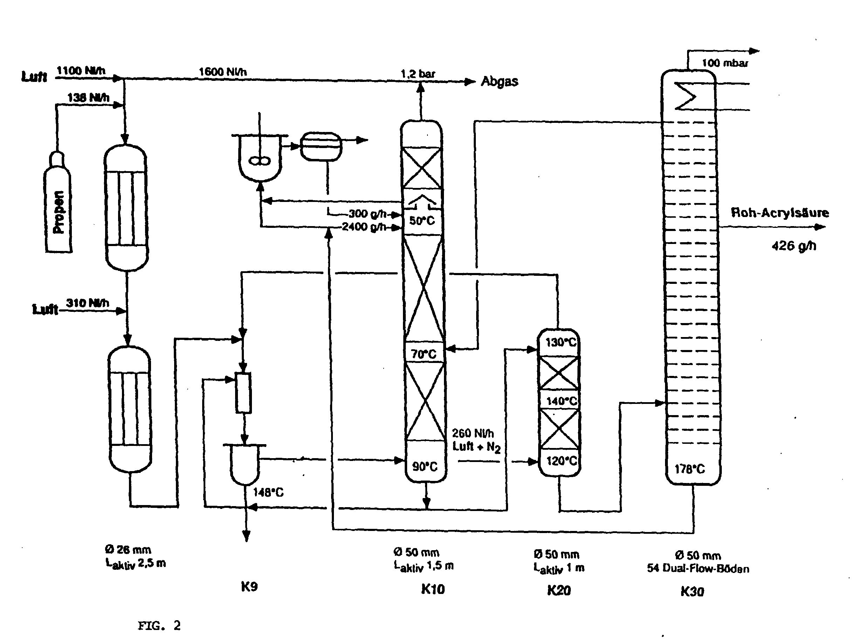

In einer Miniplant wurden stündlich 426 g Acrylsäure erzeugt. Die Verschaltung der Apparate, die geförderten Mengen und die eingestellten Betriebsparameter sind Fig. 2 zu entnehmen. In dieser Figur sind die Trennschritte wie in Fig. 1 als Apparate dargestellt gezeigt, wobei sich gleiche Bezeichnungen entsprechen. Die Oxidation von Propen mit Luft via Acrolein erfolgte in zwei hintereinandergeschalteten Reaktionsrohren mit 26 mm Durchmesser und 2,5 m Katalysatorschüttungslänge. Das erste Rohr war mit einem Schalen-Katalysator, wie er in EP 575 897 beschrieben ist, gefüllt und das zweite Reaktionsrohr enthielt einen Schalen-Katalysator, wie er in EP 609 750 beschrieben ist. Die Kolonnen K10, K20 und K30 waren verspiegelte bzw. thermostatisierte Laborkolonnen mit 50 mm Durchmesser. Der Direktkondensator K9 war ein Venturiwäscher. Die Kolonnenen K10 und K20 waren mit 5 mm-Metallwendeln gefüllt. Die Destillationskolonne K30 war mit Siebböden (Dual-Flow-Böden) aus Metall gefüllt. Die Bohrungen in den Siebböden waren so ausgeführt, daß sich Sprudelschichten ausbilden konnten.

### Dynamische Kristallisation

Die dynamische Kristallisation wurde in einem Kristallisator, wie er in DE-A- 26 06 364 (BASF) beschrieben ist durchgeführt, wobei mit einem volldurchströmten Rohr gearbeitet wurde. Die Daten des Kristallisators waren wie folgt:
- zweizügig mit je einem Rohr (Innendurchmesser 26 mm) pro Zug
- Rohrlänge 5 m
- Primärkreispumpe als Kreiselpumpe mit Drehzahlregelung
- Anlagenvolumen primärseitig ca 11 l
- Ausfrierrate ca. 45 % (Ausfrierrate = Kristallisatmasse/Masse Rohschmelze)
- 4 Stufenbehälter mit je 100 l Volumen
- Temperierung der Anlage mit Kälteanlage und 4 bar Dampf über Wärmetauscher

Die Anlage wurde über ein Prozeßleitsystem gesteuert, wobei der Programmablauf für eine Stufe wie folgt war:
1. Füllen des Primärkreises
2. Entleeren des Primärkreises und Anfrieren einer Impfschicht
3. Temperatur auf ca. 2°C unter Schmelzpunkt anheben
4. Primärkreis füllen zum Kristallisieren
5. Kristallisieren (Temperaturprogramm)
6. Nach dem Ende der Kristallisation Restschmelze abpumpen
7. Temperatur anheben zum Aufschmelzen der Kristallschicht
8. Aufgeschmolzenes Kristallisat abpumpen
9. Start einer neuen Stufe

Die Temperaturen, Drücke, Volumenströme sind abhängig von der jeweils zu fahrenden Stufe.

### Statische Schichtkristallisation

Die hierfür verwendete Anlage bestand aus einen Rohrkristallisator aus Glas, der einen Innendurchmesser von 80 mm hatte und dessen Länge 1 m betrug. Der Kristallisator wurde über einen Mantel aus Glas temperiert. Das Füllvolumen des Kristallisators betrug 2,0 bis 5,0 l (variabel). Die Temperierung der Anlage erfolgte über einen Thermostaten, wobei die Temperatursteuerung über einen Programmgeber erfolgte. Die Ausfrierrate (nach dem Schwitzen) betrug ca. 50 %. Der Programmablauf für eine Stufe war wie folgt:
1. Füllen des Kristallisators
2. Temperieren des Apparats mit Inhalt (auf ca. 1 K über Schmelztemperatur)
3. Kristallisieren (Temperaturprogramm)
4. Nach Ende des Kristallisierens Restschmelze ablassen
5. Schwitzen (Temperaturprogramm)
6. Kristallisat abschmelzen
7. Start einer neuen Stufe

Die Temperaturen sind abhängig von der jeweils zu fahrenden Stufe.

### Beispiel 1

Aus dem Seitenabzug der Destillationskolonne K30 wurden 426 g/h Roh-Acrylsäure in einer Qualität abgezogen, wie sie in Tabelle 1 unten angegeben ist.

### Beispiel 2

Die in Beispiel 1 genannte Roh-Acrylsäure wurde in einer der oben beschriebenen dynamischen Kristallisationsstufen gereinigt. Dabei wurde eine Rein-Acrylsäure in einer Reinheit von 99,95 Gew.-% mit einem Propionsäureanteil von 51 Gew.-ppm und einem Essigsäureanteil von 345 Gew.-% ppm erhalten. Der Kristallisationsrückstand dieser Reinigungsstufe wurde in drei dynamischen und zwei statischen Kristallisationsstufen weiter aufgearbeitet. Die Reinheit der auf diese Weise erhaltenen Rein-Acrylsäure ist der unten stehenden Tabelle zu entnehmen.

**Tabelle**

| **Art der Acrylsäure** | **Acrylsäure in Gew.-%** | **Propionsäure in ppm** | **Essigsäure in ppm** |
|---|---|---|---|
| Roh-Acrylsäure | 99,7 | 203 | 2000 |
| Rein-Acrylsäure | 99,95 | 51 | 345 |

Wie aus der Tabelle ersichtlich, führt die kombinierte Anwendung einer dynamischen und statischen Kristallisation zu einer hochreinen Acrylsäure. Insbesondere wurde der Propionsäuregehalt (der bei der Acrylesterherstellung zu übelriechenden Propionaten führen würde) auf ein Viertel gesenkt, was destillativ auch mit größtem Aufwand nicht möglich gewesen wäre.

## Patentansprüche

1. Verfahren zur Herstellung von Acrylsäure und/oder deren Estern, wobei das Verfahren folgende Stufen in der folgenden Reihenfolge umfasst:
(a) katalytische Gasphasenoxidation von Propen und/oder Acrolein zu Acrylsäure unter Erhalt eines gasförmigen, die Acrylsäure enthaltenden Reaktionsprodukts,
(b) Absorption des Reaktionsprodukts mit einem Lösungsmittel,
(bl) das mit Reaktionsprodukt beladene Lösungsmittel wird einer Desorption zur Abtrennung von leicht siedenden Nebenkomponenten unterzogen,
(c) Destillation des mit Acrylsäure beladenen Lösungsmittelstroms aus (bl) in einer Kolonne unter Erhalt einer Roh-Acrylsäure, die durch Seitenabzug entnommen wird, und des Lösungsmittels im Sumpf,
(d) Reinigung der Roh-Acrylsäure durch Kristallisation und
(e) gegebenenfalls Veresterung der kristallisierten Acrylsäure.

2. Verfahren nach Anspruch 1, **dadurch gekennzeichnet, dass** in Stufe (b) ein hochsiedendes Lösungsmittel verwendet wird.

3. Verfahren nach Anspruch 2, **dadurch gekennzeichnet, dass** das Lösungsmittel ein Gemisch aus Diphenylether und Biphenyl ist.

4. Verfahren nach einem der Ansprüche 1 bis 3, **dadurch gekennzeichnet, dass** es eines oder mehrere der folgenden Merkmale aufweist:
- das Reaktionsprodukt vor Stufe (b) wird abgekühlt, indem es mit dem Lösungsmittel in Kontakt gebracht und ein Teil des Lösungsmittels verdampft wird,
- die Absorption in Stufe (b) wird als Gegenstromabsorption in einer Absorptionskolonne durchgeführt, wobei das Sumpfprodukt der Absorptionskolonne vor Stufe (c) der Desorption unterzogen wird,
- das in Stufe (c) am Kopf der Destillationskolonne erhaltene Produkt wird in Stufe (b) rückgeführt,
- das in Stufe (c) am Boden der Destillationskolonne erhaltene Produkt wird der Absorptionsstufe (b) rückgeführt.

5. Verfahren nach einem der Ansprüche 1 bis 4, **dadurch gekennzeichnet, dass** in Stufe (d) eine fraktionierte Kristallisation durch Kombination von statischer und dynamischer Kristallisation eingesetzt wird.

6. Verfahren nach Anspruch 5, **dadurch gekennzeichnet, dass** in Stufe (d) der Rückstand der dynamischen Kristallisation der statischen Kristallisation zugeführt wird und das Kristallisat der statischen Kristallisation der dynamischen Kristallisation zugeführt wird, wobei vorzugsweise die dynamische Kristallisation mit einem volldurchströmten Apparat oder mittels eines Fallfilms durchgeführt wird.

7. Verfahren nach Anspruch 5 oder 6, **dadurch gekennzeichnet, dass** die dynamische und/oder statische Kristallisation in Stufe (d) als Gegenstromkristallisation durchgeführt wird.

8. Vorrichtung zur Herstellung von Acrylsäure, umfassend in der folgenden Reihenfolge
- wenigstens einen Reaktor für eine katalytische Gasphasenreaktion,
- eine Absorptionskolonne,
- eine Desorptionskolonne,
- eine Destillationskolonne mit Seitenabzug, durch den Acrylsäure abgezogen wird,
- wenigstens einen dynamischen Kristallisator und wenigstens einen statischen Kristallisator und
- wenigstens einen Behälter zur Zwischenlagerung.

9. Vorrichtung nach Anspruch 8, **dadurch gekennzeichnet, dass** sie eines oder mehrere der folgenden Merkmale aufweist:
- zwischen dem Reaktor für die katalytische Gasphasenreaktion und der Absorptionskolonne ist ein Direktkondensator zur Teilverdampfung von Lösungsmittel vorgesehen,
- die Absorptionskolonne ist als Gegenstromabsorptionskolonne ausgebildet,
- die Destillationskolonne ist mit Siebböden ausgestattet,
- der dynamische und/oder statische Kristallisator ist als Wärmetauscher ausgebildet.

## Claims

1. A process for preparing acrylic acid and/or esters, comprising the following the steps in the following order:
(a) catalytic gas phase oxidation of propene and/or acrolein to acrylic acid to obtain a gaseous reaction product comprising acrylic acid,
(b) solvent absorption of the reaction product,
(b1) the solvent loaded with reaction product is subjected to a desorption to remove low boiling secondary components,
(c) distillation of the solvent stream loaded with acrylic acid from (b1) in a column to obtain a crude acrylic acid, which is removed as a sidestream, and the solvent,
(d) purification of the crude acrylic acid by crystallization and
(e) optionally esterification of the crystalline acrylic acid.

2. A process as claimed in claim 1, wherein a high boiling solvent is used in step (b).

3. A process as claimed in claim 2, wherein the solvent is a mixture of diphenyl ether and biphenyl.

4. A process as claimed in one of claims 1 to 3, which has one or more of the following features:
- the reaction product is cooled ahead of step (b) by contacting it with the solvent and evaporating part of the solvent,
- the absorption of step (b) is carried out as countercurrent absorption in an absorption column, the bottom product of the absorption column being subjected to the desorption ahead of step (c),
- the product obtained at the top of the distillation column in step (c) is recycled into step (b),
- the product obtained at the base of the distillation column in step (c) is recycled into the absorption step (b).

5. A process as claimed in any of claims 1 to 4, wherein step (d) utilizes a fractionate crystallization through combination of static and dynamic crystallization.

6. A process as claimed in claim 5, wherein, in step (d), the dynamic crystallization residue is subjected to a static crystallization and the static crystallization product is subjected to dynamic crystallization, the dynamic crystallization preferably being carried out with a full flow apparatus or by means of a falling film.

7. A process as claimed in claim 5 or 6, wherein the dynamic and/or static crystallizations of step (d) are carried out as countercurrent crystallizations.

8. Apparatus for preparing acrylic acid, comprising in the following order:
- at least one reactor for a catalytic gas phase reaction,
- an absorption column,
- a desorption column,
- a distillation column with a sidestream by which the acrylic acid is withdrawn,
- at least one dynamic crystallizer and at least one static crystallizer, and
- at least one intermediate storage vessel.

9. Apparatus as claimed in claim 8, which has one or more of the following features:
- a direct condenser for the partial evaporation of solvent is provided between the catalytic gas phase reactor and the absorption column,
- the absorption column is constructed as a countercurrent absorption column,
- the distillation column is equipped with sieve plates,
- the dynamic and/or static crystallizers are constructed as heat exchangers.

## Revendications

1. Procédé de préparation d'acide acrylique et /ou de ses esters, le procédé comprenant les étapes suivantes dans l'ordre suivant:
(a) Oxydation catalytique en phase gazeuse de propène et/ou d'acroléine en acide acrylique, avec obtention d'un produit de réaction gazeux contenant l'acide acrylique,
(b) absorption du produit de réaction au moyen d'un solvant,
(b1) le solvant chargé du produit de réaction est soumis à une désorption afin de séparer les sous-produits à bas point d'ébullition,
(c) distillation du flux de solvant chargé d'acide acrylique obtenu en (b1) dans une colonne, avec obtention d'un acide acrylique brut, prélevé par soutirage latéral, et du solvant en fond de colonne,
(d) épuration de l'acide acrylique brut par cristallisation et
(e) estérification éventuelle de l'acide acrylique purifié.

2. Procédé selon la revendication 1, **caractérisé en ce que** l'on utilise dans l'étape (b) un solvant à haut point d'ébullition.

3. Procédé selon la revendication 2, **caractérisé en ce que** le solvant est un mélange d'éther diphénylique et de biphényle.

4. Procédé selon l'une des revendications 1 à 3, **caractérisé en ce qu'**il présente une ou plusieurs des caractéristiques suivantes:
- le produit de réaction est refroidi avant l'étape (b), **en ce qu'**il est mis en contact avec le solvant et qu'une partie du solvant est évaporée,
- dans l'étape (b), l'absorption est entreprise en mode d'absorption à contre-courant dans une colonne d'absorption, et le produit de fond de colonne dé la colonne d'absorption est soumis à la désorption avant l'étape (c)
- on recycle dans l'étape (b) le produit recueilli en tête de la colonne de distillation à l'étape (c),
- on recycle dans l'étape d'absorption (b) le produit recueilli dans l'étape (c) au bas de la colonne de distillation.

5. Procédé selon l'une des revendications 1 à 4, **caractérisé en ce que** l'on met en oeuvre, dans l'étape (d), une cristallisation fractionnée par combinaison de cristallisation statique et dynamique.

6. Procédé selon la revendication 5, **caractérisé en ce que** l'on envoie dans l'étape (d) le résidu de la cristallisation dynamique et de la cristallisation statique et le produit cristallisé de la cristallisation statique et de la cristallisation dynamique, la cristallisation dynamique étant de préférence entreprise dans un appareillage à passage intégral ou en mode à film descendant.

7. Procédé selon la revendication 5 ou 6, **caractérisé en ce que** la cristallisation dynamique et/ou statique est entreprise dans l'étape (d) en tant que cristallisation à contre-courant.

8. Dispositif de préparation d'acide acrylique comprenant, dans l'ordre:
- au moins un réacteur pour la réaction catalytique en phase gazeuse,
- une colonne d'absorption,
- une colonne de désorption,
- une colonne de distillation avec un soutirage latéral, par lequel l'acide acrylique est recueilli,
- au moins un cristalliseur dynamique et au moins un cristalliseur statique et
- au moins un réservoir d'entreposage intermédiaire.

9. Dispositif selon la revendication 8, **caractérisé en ce qu'**il présente une ou plusieurs des caractéristiques suivantes:
- entre le réacteur de réaction catalytique en phase gazeuse et la colonne d'absorption est prévu un condenseur direct pour l'évaporation partielle du solvant,
- la colonne d'absorption est configurée en colonne d'absorption à contre-courant,
- le colonne de distillation est équipée d'un fond à tamis,
- le cristalliseur dynamique et/ou statique est configuré en échangeur de chaleur.
